# EUROPEAN PATENT APPLICATION

(11) **EP 4 831 151 A1**
(43) Date of publication of application: **28.10.2026**
(21) Application number: 26165468.5
(22) Date of filing: 17.03.2026
(51) Int. Cl.: C07C 29/151, C07C 29/80, C07C 31/04

(54) **METHANOL PRODUCTION SYSTEM**

(30) Priority: 24.04.2025 KR 20250053885
(71) Applicant: Doosan Enerbility Co., Ltd., Seongsan-gu Changwon-si 51711 (KR)
(72) Inventor: Kim, Hyun Min, 16868 Yongin-si, Gyeonggi-do (KR); Sohn, Geun, 13607 Seongnam-si, Gyeonggi-do (KR); Bae, Seong Hee, 18475 Hwaseong-si, Gyeonggi-do (KR)
(74) Representative: BCKIP Part mbB

(57) **Abstract**

A methanol production system is provided. The methanol production system includes a methanol production reactor configured to produce methanol from a feed gas comprising carbon dioxide and hydrogen, a gas-liquid separator configured to separate an unreacted gas from a reaction product produced in the methanol production reactor, and a distillation column configured to distill methanol from the reaction product from which the unreacted gas has been separated by the gas-liquid separator, wherein at least a portion of wastewater discharged from the distillation column after the methanol is distilled is supplied to the methanol production reactor as cooling water.

## Description

This application claims priority to Korea Patent Application No. 10-2025-0053885, filed on April 24, 2025.

### FIELD

Apparatuses and methods consistent with exemplary embodiments relate to a methanol production system, and more particularly, to a methanol production system capable of achieving a compact equipment configuration by utilizing wastewater separated after methanol production as cooling water for temperature control of a methanol production reactor, thereby eliminating the need for separate purification facilities for wastewater treatment and reducing the amount of wastewater produced.

### BACKGROUND

In conventional methanol production systems, wastewater (process water) separated after methanol production is purified through a separate purification facility to remove compounds such as hydro alcohols, esters, dimethyl ether (DME), and ketones generated during the reaction. The wastewater (process water) purified into deionized (DI) water through separate purification facilities is then used for water electrolysis. The eco-friendly hydrogen produced through water electrolysis, powered by renewable energy, is then utilized in methanol production.

However, these conventional methanol production systems face significant integration challenges for offshore applications. The reliance on land-based water electrolysis and the spatial requirements for complex purification equipment typically restrict these systems to onshore facilities. In addition, conventional systems often necessitate the transportation of methanol from onshore production sites to marine vessels. This logistical requirement results in increased carbon emissions during the transportation process and reduces the overall efficiency of the fuel supply chain for marine applications.

### SUMMARY

Aspects of one or more exemplary embodiments provide a methanol production system capable of achieving a compact equipment configuration by utilizing wastewater separated after methanol production as cooling water for temperature control of a methanol production reactor, thereby eliminating the need for separate purification facilities for wastewater treatment and reducing the amount of wastewater produced.

Additional aspects will be set forth in part in the description which follows and, in part, will become apparent from the description, or may be learned by practice of the exemplary embodiments.

According to an aspect of an exemplary embodiment, there is provided a methanol production system including: a methanol production reactor configured to produce methanol from a feed gas comprising carbon dioxide and hydrogen; a gas-liquid separator configured to separate an unreacted gas from a reaction product produced in the methanol production reactor; and a distillation column configured to distill methanol from the reaction product from which the unreacted gas has been separated by the gas-liquid separator, wherein at least a portion of wastewater discharged from the distillation column after the methanol is distilled is supplied to the methanol production reactor as cooling water.

The at least a portion of the wastewater may be supplied to the methanol production reactor in an unpurified state.

The methanol production system may further include a compressor configured to compress the feed gas and supply the compressed feed gas to the methanol production reactor.

The compressor may compress the feed gas to an operating pressure within a range of 30 bar to 70 bar prior to delivery to the methanol production reactor.

The unreacted gas separated in the gas-liquid separator may be supplied to the compressor to be compressed together with the feed gas or to the methanol production reactor at a pressure substantially equal to the reaction pressure.

The methanol production system may further include a direct air capture (DAC) device configured to extract carbon dioxide from an ambient atmospheric environment, wherein the DAC device may be fluidly coupled to the compressor such that the extracted carbon dioxide is supplied to the compressor as a constituent of the feed gas.

The hydrogen may be generated via water electrolysis, and the generated hydrogen may be supplied to the compressor as a constituent of the feed gas.

The methanol production system may further include a heat exchanger configured to preheat the feed gas or the unreacted gas separated in the gas-liquid separator by using reaction heat generated in the methanol production reactor.

A heat exchange medium may be configured to circulate to recover thermal energy from the methanol production reactor and deliver the thermal energy to the heat exchanger to preheat the feed gas or the unreacted gas.

The methanol production system may be disposed on an offshore platform.

The methanol production system may further include a purifier configured to desalinate seawater to produce a purified water stream, wherein the purified water stream may be electrolyzed to generate the hydrogen.

The purifier may include at least one of a reverse osmosis (RO) device and a filtration membrane device configured to remove salts and organic contaminants from the seawater or the wastewater.

The methanol production system may further include a byproduct measuring device fluidly coupled to a discharge of the distillation column and configured to determine a concentration of organic byproducts within the wastewater, wherein, in response to the byproduct measuring device determining that the concentration of organic byproducts is equal to or less than a predetermined threshold value, the wastewater is supplied to the purifier for desalination.

In response to the byproduct measuring device determining that the concentration of organic byproducts exceeds the predetermined threshold value, the wastewater is diverted to a wastewater storage tank and bypassed around the purifier.

A volumetric portion of the wastewater comprising 30% to 50% of the wastewater discharged from the distillation column may be recirculated to the methanol production reactor as the cooling water.

According to one or more exemplary embodiments, the wastewater separated after methanol production is used as cooling water for temperature control in the methanol production reactor, thereby eliminating the need for a separate purification facility for wastewater treatment and reducing the amount of wastewater generated. Accordingly, the overall process steps, including water electrolysis, can be reduced and a compact equipment configuration can be achieved, making the system applicable to an offshore platform.

As described above, applying the methanol production system to an offshore platform allows direct use of carbon generated in the marine environment, and after methanol production, marine methanol can be supplied to ships through coastal supply or bunkering without generating additional carbon emissions.

In addition, when applying the methanol production system to an offshore platform, seawater can be purified into distilled water and used for water electrolysis, and in some cases, if the byproducts in the wastewater are determined to be equal to or less than a predetermined threshold criterion, the wastewater may be purified and used for electrolysis.

### BRIEF DESCRIPTION OF THE DRAWINGS

The above and other will be more clearly understood from the following description of the exemplary embodiments with reference to the accompanying drawings, in which:
FIG. 1 is a schematic diagram illustrating a methanol production system according to a first exemplary embodiment;
FIG. 2 is a schematic diagram illustrating a methanol production system according to a second exemplary embodiment; and
FIG. 3 is a schematic diagram illustrating a methanol production system according to a third exemplary embodiment.

### DETAILED DESCRIPTION

Various modifications and various embodiments will be described with reference to accompanying drawings so that those skilled in the art can easily carry out the disclosure. It should be understood, however, that the various embodiments are not for limiting the scope of the disclosure to the specific embodiments, but they should be interpreted to include all modifications, equivalents or alternatives of the embodiments included within the spirit and scope disclosed herein.

The terms described below are for the purpose of describing specific embodiments only, and are not intended to limit the scope of the present disclosure. As used herein, the singular forms "a", "an" and "the" are intended to include the plural forms as well, unless the context clearly indicates otherwise. In the disclosure, terms such as "comprises", "includes", or "have/has" should be construed as designating that there are such features, integers, steps, operations, components, parts, and/or combinations thereof, and do not exclude the presence or possibility of adding of one or more of other features, integers, steps, operations, components, parts, and/or combinations thereof.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Terms such as "first," "second," and so on may be used to describe a variety of elements, but the elements should not be limited by these terms. The terms are used simply to distinguish one element from other elements. The use of such ordinal numbers should not be construed as limiting the meaning of the term. For example, the components associated with such an ordinal number should not be limited in the order of use, placement order, or the like. If necessary, each ordinal number may be used interchangeably.

The terms, such as 'part' or 'module', etc., should be understood as a unit that performs at least one function or operation and that may be embodied as hardware, software, or a combination thereof. With respect to an element described as a "unit" or "module", two or more elements may be combined into a single element, or a single element may be divided into two or more elements according to the subdivided functions. Also, each element described below may additionally perform some or all of functions performed by other elements, in addition to its main functions, and some of the main functions of each element may be performed entirely by other components.

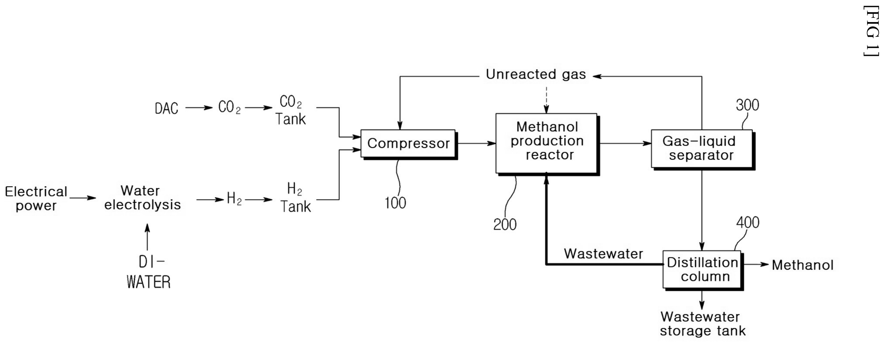

Hereinafter, a methanol production system according to a first exemplary embodiment will be described in detail with reference to FIG. 1.

Referring to FIG. 1, the methanol production system comprises a compressor 100, a methanol production reactor 200, a gas-liquid separator 300, and a distillation column 400. In one or more exemplary embodiments, any or all components may be arranged in multiple stages (e.g., a multi-stage compressor or a series of reactors) to optimize throughput or reaction efficiency.

The methanol production reactor 200 produces methanol using a feed gas containing carbon dioxide (CO₂) and hydrogen (H₂).

The compressor 100 compresses the feed gas and supplies the compressed feed gas to the methanol production reactor 200. In one or more exemplary embodiments, the compressor 100 is configured to increase a pressure of the feed gas to a range of approximately 30 bar to 70 bar.

The gas-liquid separator 300 separates unreacted gas from the reaction product generated in the methanol production reactor 200. The reaction product generated in the methanol production reactor 200 includes unreacted gas, methanol, water, and various byproducts.

The unreacted gas separated by the gas-liquid separator 300 is supplied to the compressor 100 for reuse in methanol production process, allowing the unreacted gas to be repressurized in combination with the feed gas. Alternatively, the unreacted gas separated by the gas-liquid separator 300 may be supplied to the methanol production reactor 200 for reuse, depending on the pressure differential within the system.

The distillation column 400 distills methanol from the reaction product from which the unreacted gas is separated in the gas-liquid separator 300. In one or more exemplary embodiments, the purified methanol distilled by the distillation column 400 may serve as a feedstock for further synthesis. For example, the system may be configured to further process the methanol into dimethyl ether (DME), sustainable aviation fuel (SAF), or other synthetic hydrocarbons.

The system is configured to utilize at least a portion of the wastewater discharged from the distillation column 400 as cooling medium for the methanol production reactor 200. During the methanol production process, an exothermic reaction occurs within the methanol production reactor 200, generating significant reaction heat. To manage this thermal load, a cooling supply line fluidly couples a wastewater outlet of the distillation column 400 to a thermal interface - such as a cooling jacket or heat exchanger - integrated with the methanol production reactor 200. The wastewater is circulated through the thermal interface to absorb the reaction heat, thereby regulating the operating temperature of the methanol production reactor 200.

The wastewater is not purified before being supplied to the methanol production reactor 200. In addition to water, the wastewater contains byproducts (e.g., hydro alcohols, esters, DME, ketones, etc.). However, unlike conventional systems, the wastewater is not used for water electrolysis. Instead, the wastewater is utilized as cooling water to control the temperature of the methanol production reactor 200 (e.g., to maintain isothermal conditions), eliminating the need for a separate purification facility to remove the byproducts. Therefore, this can contribute to reducing the load on the water treatment system.

By utilizing the unpurified wastewater as a cooling medium, the system obviates the necessity for a dedicated wastewater purification facility. This integration reduces the amount of wastewater discharge and simplifies the overall process flow by eliminating the intermediate purification steps associated with water electrolysis feedstocks. Consequently, the system achieves a compact equipment configuration and a reduced spatial footprint. These structural efficiencies enable the methanol production system to be deployed across a variety of installations, including both onshore facilities and offshore platforms where deck space and weight considerations are critical. The reduction in process complexity further enhances the reliability and operational efficiency of the system in remote marine environments.

The following describes the methanol production system of the present disclosure based on installation on an offshore platform. The offshore platform of marine floating plants may include a fixed structure, a floating structure, or a coastal-connected facility, depending on the specific environmental and regulatory requirements of the deployment site. When the methanol production system is integrated with an offshore platform, the purified methanol product is configured for direct supply to marine vessels via coastal supply lines or bunkering interfaces. This localized production and distribution architecture minimizes or eliminates the requirement for long-distance transport of methanol from onshore facilities, thereby reducing carbon emissions.

The remaining portion of the wastewater discharged from the distillation column 400 which is not supplied to the methanol production reactor 200 is stored in a wastewater storage tank for subsequent processing or disposal. At least a portion of the wastewater is recycled as cooling water, thereby minimizing the volume of the wastewater storage tank.

In one or more exemplary embodiments, approximately 30% to 50% of the wastewater discharged from the distillation column 400 is recycled to the methanol production reactor 200 as cooling water. For example, when producing approximately 300,000 TPY (tons per year) of methanol (roughly 43,611 kg/hr), the system generates approximately 170,000 TPY (roughly 23,933 kg/hr) of wastewater. In a configuration in which the methanol production reactor 200 comprises a boiling water reactor (BWR), the cooling requirement for thermal regulation is approximately 51,500 kg/hr. Under these operational parameters, approximately 40% of the generated wastewater is utilized as cooling water for reactor temperature control.

In one or more exemplary embodiments, the system may further include a direct air capture (DAC) device configured to sequester carbon dioxide from the ambient marine environment. Unlike conventional systems that may require the transport of carbon dioxide from onshore facilities, the integrated DAC device allows for the direct, in-situ utilization of atmospheric or marine carbon, thereby eliminating the logistical requirements and carbon emissions associated with carbon dioxide transportation. The carbon dioxide sequestered by the DAC device can be stored in a carbon dioxide storage tank and supplied to the compressor 100.

In one or more exemplary embodiments, hydrogen is produced by water electrolysis of distilled water (DI-water). The power required for electrolysis may be supplied from renewable energy, energy storage systems (ESS), external power sources, or a combination thereof. Because methanol production requires a higher molar ratio of hydrogen to carbon dioxide, if the hydrogen produced by electrolysis is insufficient, additional hydrogen can be used from nuclear-powered electrolysis, steam methane reforming, or other sources. The hydrogen produced by electrolysis can be stored in a hydrogen storage tank and supplied to the compressor 100.

The methanol production system of the present disclosure is configured to implement a substantially carbon-neutral synthesis process. The methanol produced by the system is characterized as e-methanol (electrolytic methanol), as it is produced from carbon dioxide sequestered by the DAC device and hydrogen generated through water electrolysis powered by renewable energy.

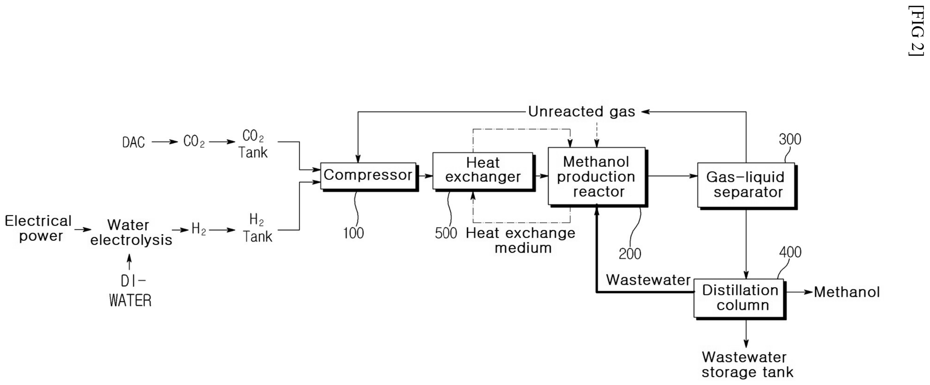

A methanol production system according to a second exemplary embodiment will be described with reference to FIG. 2.

Referring to FIG. 2, the methanol production system includes a compressor 100, a methanol production reactor 200, a gas-liquid separator 300, a distillation column 400, and a heat exchanger 500. The methanol production system of FIG. 2 is substantially similar to the methanol production system of FIG. 1 except for the configuration of the heat exchanger 500. For the sake of brevity, only the features of the heat exchanger 500 that differ from the first exemplary embodiment will be described.

The heat exchanger 500 preheats the feed gas or the unreacted gas separated in the gas-liquid separator 300 using the reaction heat generated in the methanol production reactor 200. In this embodiment, the heat exchanger 500 is fluidly disposed between the compressor 100 and the methanol production reactor 200 to preheat the feed gas supplied to the methanol production reactor 200.

In one or more exemplary embodiments, the heat exchanger is selectively positioned within the recycle loop to optimize the thermal profile of the unreacted gas. For example, the heat exchanger 500 may be fluidly disposed between the gas-liquid separator 300 and the compressor 100 to preheat the unreacted gas prior to compression. Alternatively, the heat exchanger 500 may be fluidly disposed between the gas-liquid separator 300 and the methanol production reactor 200 to preheat the unreacted gas before it is reintroduced into the synthesis reaction.

In this embodiment, a heat exchange medium (e.g., a thermal transfer fluid or boiler feed water) circulates in a closed-loop thermal circuit between the methanol production reactor 200 and the heat exchanger 500. The methanol production reactor 200 serves as a heat source, in which the heat exchange medium absorbs the reaction heat generated during methanol synthesis. The heated medium is then directed to the heat exchanger 500, where it undergoes indirect heat exchange with the feed gas (or unreacted gas). Following the thermal transfer, the cooled heat exchange medium is circulated back to the methanol production reactor 200 to repeat the cycle. This integrated thermal management architecture effectively recovers and utilizes internal process heat, thereby minimizing the requirement for external heating utilities and enhancing the overall thermal efficiency of the system.

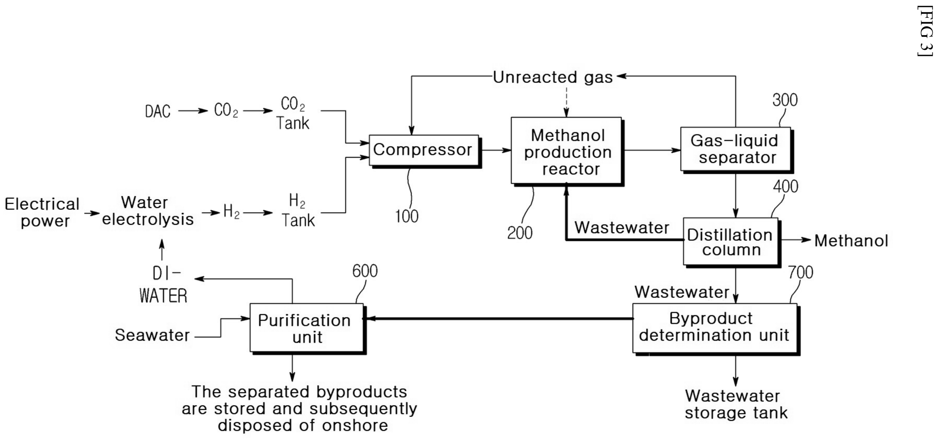

A methanol production system according to a third exemplary embodiment will be described with reference to FIG. 3.

Referring to FIG. 3, the methanol production system includes a compressor 100, a methanol production reactor 200, a gas-liquid separator 300, a distillation column 400, a purifier 600, and a byproduct measuring device 700. The methanol production system of FIG. 3 is substantially similar in arrangement to the methanol production system of FIG. 1 except for the configuration of the purifier 600 and the byproduct measuring device 700. For the sake of brevity, only the features of the purifier 600 and the byproduct measuring device 700 that differ from the first exemplary embodiment will be described.

In an embodiment configured for an offshore platform, the methanol production system is adapted to produce hydrogen via the electrolysis of seawater. To facilitate this, the system comprises the purifier 600 configured to treat seawater to produce distilled or deionized water suitable for electrolysis. The purifier 600 is specifically configured for the removal of salts and mineral solutes and is structurally distinct from the organic-byproduct purification facilities typically required for wastewater treatment. Because the purifier 600 focuses on ionic separation rather than complex organic byproduct removal, it achieves a reduced spatial footprint within the integrated system. For example, the purifier 600 may include a reverse osmosis (RO) device, a nanofiltration membrane device, or a combination thereof. The byproducts generated by the purifier 600 may be stored in a storage tank for subsequent onshore disposal or, where environmentally permissible, discharged.

Furthermore, the portion of wastewater discharged from the distillation column 400 that is not supplied to the methanol production reactor 200 is supplied to the byproduct measuring device 700. The byproduct measuring device 700 is configured to perform a quantitative analysis to determine whether the concentration of byproducts within the wastewater is equal to or less than a predetermined threshold criterion. The predetermined threshold criterion may be dynamically adjusted based on localized environmental regulations, discharge permits, or specific operational requirements of the offshore platform. For example, the byproduct measuring device 700 may be configured to assess the concentration of byproducts in parts-per-million (ppm) units.

When producing hydrogen through seawater electrolysis, chloride ions present in the seawater may undergo oxidation at the anode (electrolytic oxidation electrode) resulting in the generation of other byproducts such as chlorine. After identifying the byproducts, the wastewater may be purified as needed and reused in the process stages.

If the byproduct measuring device 700 determines that the concentration of byproducts within the wastewater is equal to or less than the predetermined threshold criterion, the wastewater is supplied to the purifier 600. In this configuration, the wastewater is purified together with seawater and can be used for electrolysis, thereby increasing hydrogen production and further reducing the amount of wastewater.

If the byproduct measuring device 700 determines that the concentration of byproducts within the wastewater exceed the predetermined threshold criterion, the wastewater is not supplied to the purifier 600 but is supplied to and stored in a wastewater storage tank.

In one or more exemplary embodiments, a volumetric portion of the wastewater comprising 30% to 50% of the wastewater discharged from the distillation column 400 is recycled to the methanol production reactor 200 as cooling water. Recycling less than 30% of the wastewater may result in a mass flow rate insufficient for effective heat removal from the methanol production reactor 200, while recycling more than 50% of the wastewater may result in an accumulation of thermal energy that exceeds the cooling capacity of a heat sink. For example, a remaining portion of the wastewater not used as the cooling water is fluidly directed to the purifier 600.

While one or more exemplary embodiments have been described with reference to the accompanying drawings, it will be apparent to those skilled in the art that various variations and modifications may be made by adding, changing, or removing components without departing from the spirit and scope of the disclosure as defined in the claims, and these variations and modifications fall within the spirit and scope of the disclosure as defined in the appended claims. Therefore, the description of the exemplary embodiments should be construed in a descriptive sense and not to limit the scope of the claims, and many alternatives, modifications, and variations will be apparent to those skilled in the art.

## Claims

1. A methanol production system comprising:
a methanol production reactor configured to produce methanol from a feed gas comprising carbon dioxide and hydrogen;
a gas-liquid separator configured to separate an unreacted gas from a reaction product produced in the methanol production reactor; and
a distillation column configured to distill methanol from the reaction product from which the unreacted gas has been separated by the gas-liquid separator,
wherein at least a portion of wastewater discharged from the distillation column after the methanol is distilled is supplied to the methanol production reactor as cooling water.

2. The methanol production system according to claim 1,
wherein the at least a portion of the wastewater is supplied to the methanol production reactor in an unpurified state.

3. The methanol production system according to claim 1, further comprising:
a compressor configured to compress the feed gas and supply the compressed feed gas to the methanol production reactor.

4. The methanol production system according to claim 3,
wherein the compressor compresses the feed gas to an operating pressure within a range of 30 bar to 70 bar prior to delivery to the methanol production reactor.

5. The methanol production system according to claim 3,
wherein the unreacted gas separated in the gas-liquid separator is supplied to the compressor to be compressed together with the feed gas or to the methanol production reactor at a pressure substantially equal to the reaction pressure.

6. The methanol production system according to claim 1, further comprising:
a direct air capture (DAC) device configured to extract carbon dioxide from an ambient atmospheric environment,
wherein the DAC device is fluidly coupled to the compressor such that the extracted carbon dioxide is supplied to the compressor as a constituent of the feed gas.

7. The methanol production system according to claim 1,
wherein the hydrogen is generated via water electrolysis, and the generated hydrogen is supplied to the compressor as a constituent of the feed gas.

8. The methanol production system according to claim 1, further comprising:
a heat exchanger configured to preheat the feed gas or the unreacted gas separated in the gas-liquid separator by using reaction heat generated in the methanol production reactor.

9. The methanol production system according to claim 8,
wherein a heat exchange medium is configured to circulate to recover thermal energy from the methanol production reactor and deliver the thermal energy to the heat exchanger to preheat the feed gas or the unreacted gas.

10. The methanol production system according to claim 2,
wherein the methanol production system is disposed on an offshore platform.

11. The methanol production system according to claim 10, further comprising:
a purifier configured to desalinate seawater to produce a purified water stream,
wherein the purified water stream is electrolyzed to generate the hydrogen.

12. The methanol production system according to claim 11,
wherein the purifier comprises at least one of a reverse osmosis (RO) device and a filtration membrane device configured to remove salts and organic contaminants from the seawater or the wastewater.

13. The methanol production system according to claim 11, further comprising:
a byproduct measuring device fluidly coupled to a discharge of the distillation column and configured to determine a concentration of organic byproducts within the wastewater,
wherein, in response to the byproduct measuring device determining that the concentration of organic byproducts is equal to or less than a predetermined threshold value, the wastewater is supplied to the purifier for desalination.

14. The methanol production system according to claim 13,
wherein, in response to the byproduct measuring device determining that the concentration of organic byproducts exceeds the predetermined threshold value, the wastewater is diverted to a wastewater storage tank and bypassed around the purifier.

15. The methanol production system according to claim 1,
wherein a volumetric portion of the wastewater comprising 30% to 50% of the wastewater discharged from the distillation column is recirculated to the methanol production reactor as the cooling water.
